# FASCICULE DE BREVET EUROPEEN

(11) **EP 0 803 490 B1**
(45) Date de publication et mention de la délivrance du brevet: **16.08.2000**
(21) Numéro de dépôt: 97400920.1
(22) Date de dépôt: 23.04.1997
(51) Int. Cl.: C07C 2/30, C07C 11/08

(54) **Procédé amélioré de conversion de l'éthylène en butène-1 avec utilisation d'additifs à base de sels d'ammonium quaternaire**
Verbessertes Verfahren zur Umwandlung von Ethylen in 1-Buten unter Verwendung von Additiven die auf quaternären Ammoniumsalzen basieren
Improved process for the conversion of ethylene to 1-butene using additives which are based on quaternary ammonium salts

(30) Priorité: 26.04.1996 FR 9605400
(43) Date de publication de la demande: 29.10.1997
(73) Titulaire: INSTITUT FRANCAIS DU PETROLE, 92500 Rueil Malmaison (FR); Saudi Basic Industries Corporation (SABIC), Riyadh 11422 (SA)
(72) Inventeur: Commereuc, Dominique, 92190 Meudon (FR); Hugues, François, 69390 Vernaison (FR); Chauvin, Yves, 78230 Le Pecq (FR); Glaize, Yves, 69360 Saint Symphorien d'Ozon (FR)
(74) Mandataire: Andreeff, François

(56) Documents cités:
- US-A- 4 532 370
- US-A- 4 615 998

## Description

L'objet de la présente invention est un procédé amélioré de synthèse du butène-1 par dimérisation de l'éthylène, grâce à l'utilisation d'additifs à base de sels d'ammonium quaternaire.

Dans le procédé de dimérisation de l'éthylène en butène-1 au moyen d'un catalyseur homogène obtenu par l'interaction d'un mélange préformé d'un titanate d'alkyle et d'un éther avec un composé d'aluminium de formule AlR₃ ou AlR₂H tel que décrit dans les brevets US 4 532 370 et 4 615 998, il se forme de petites quantités de polymère solide qui se déposent sur la surface du réacteur et des tubes d'échangeurs de chaleur et qui sont très nuisibles à la bonne marche du procédé car elles réduisent les transferts de chaleur et nécessitent l'arrêt fréquent du réacteur afin de les éliminer.

Il a maintenant été trouvé que si la réaction de dimérisation est conduite en présence d'additifs composés par des sels d'ammonium quaternaire, la quantité de polymère solide sous-produit est réduite et son adhérence aux parois du réacteur et des échangeurs est considérablement diminuée.

L'invention a ainsi pour objet un procédé amélioré de conversion de l'éthylène en butène-1, dans lequel, dans une enceinte réactionnelle, on met l'éthylène en contact avec une solution d'un catalyseur obtenu par l'interaction d'au moins un titanate d'alkyle avec un composé d'aluminium de formule AlR₃ ou AlR₂H, chacun des restes R étant un radical hydrocarbyle, et en présence d'au moins un additif choisi dans le groupe formé par les sels d'ammonium quaternaire.

Les éléments de la solution de catalyseur sont décrits dans les brevets US 4.532.370 et US 4.615.998 dont les enseignements sont ci-inclus.

Les titanates d'alkyle utilisés dans l'invention répondent à la formule générale Ti(OR')₄ dans laquelle R' est un radical alkyle linéaire ou ramifié comportant de préférence de 2 à 8 atomes de carbone. On peut citer par exemple le titanate de tétraéthyle, le titanate de tétraisopropyle, le titanate de tétra-n-butyle, le titanate de tétra-éthyl-2-hexyle.

De façon particulièrement avantageuse, la solution de catalyseur résulte de l'interaction d'un mélange préformé d'au moins un titanate d'alkyle et d'au moins un éther, avec au moins un composé d'aluminium tel que défini ci-dessus. Il a été observé que l'addition d'éther permet de diminuer la quantité de polymère formé lors de la conversion.

Les éthers utilisables peuvent être des monoéthers ou des polyéthers. On peut utiliser par exemple le diéthyléther, le diisopropyléther, le dibutyléther, le méthyl-t-butyléther, le tétrahydrofurane, le dioxane-1,4, le dihydropyrane, l'éther diméthylique de l'éthylèneglycol. Les éthers préférés sont le tétrahydrofurane et / ou le dioxane-1,4. Ils sont utilisés seuls ou en mélange.

Lesdits éthers sont utilisés dans un rapport molaire de 0 à 10, avantageusement de 0,1 à 10 ou de 0,5 à 10, de préférence de 1 à 5, plus particulièrement 2 à 4 moles d'éther par mole de composé du titane. Sans être lié à aucune théorie, on peut penser que l'éther se complexe sur le titane, lui permettant ainsi d'être hexacoordiné. Si on met en oeuvre l'éther dans des rapports supérieurs à 10 moles d'éther par mole de titane, par exemple 20 et au delà, ou s'il est utilisé comme solvant de la réaction, on observe que la réaction est considérablement ralentie et que sa sélectivité est moins bonne, et même, dans certains cas, que la réaction ne se produit plus du tout.

Lesdits composés de l'aluminium utilisés pour préparer le catalyseur sont représentés par la formule générale AlR₃ ou AlR₂H dans laquelle R est un radical hydrocarbyle, de préférence alkyle, comprenant de 2 à 6 atomes de carbone. Le composé AlR₃ est préféré. On peut citer à titre d'exemple le triéthylaluminium, le tripropylaluminium, le tri-iso-butylaluminium, le trihexylaluminium.

Les composants du catalyseur peuvent être mis en contact dans un hydrocarbure et / ou dans le butène-1 produit de dimérisation et / ou dans un, ou les, sous-produit de la réaction tels que les hexènes, de préférence en présence d'éthylène. Le rapport molaire entre le composé de l'aluminium et celui du titane est d'environ 1 : 1 à 20 : 1 et de préférence de environ 2 : 1 à 5:1. La concentration du titane dans la solution ainsi préparée est avantageusement comprise entre 10⁻⁴ et 0,5 mole par litre et de préférence entre 2.10⁻³ et 0,1 mole par litre.

La température à laquelle se fait la préparation du catalyseur est habituellement comprise entre -10 et +80 °C, de préférence entre -10 et +45° C. Quand de l'éthylène est présent dans le milieu, sa quantité correspond de préférence à la saturation de la solution à la température considérée et à la pression choisie, 1 bar ou plus. La solution de catalyseur ainsi obtenue peut être utilisée telle quelle ou elle peut être diluée par addition des produits de la réaction.

Les sels d'ammonium quaternaire utilisés selon l'invention répondent à la formule générale [(R₁R₂R₃R₄)N⁺]X⁻ dans laquelle R₁, R₂, R₃ et R₄ sont des radicaux hydrocarbyle, identiques ou différents, par exemple des radicaux alkyle, cycloalkyle, aryle, cycloalkyle ou aryle substitués par un groupe alkyle, comprenant de 1 à 30 atomes de carbone, et X est un anion monovalent, par exemple un halogénure ou un hydroxyde. On peut citer à titre d'exemple le chlorure de tétraéthylammonium, le bromure de tétraéthylammonium, le chlorure de triméthyl-cétylammonium, le bromure de triméthyl-cétylammonium, le chlorure de diméthyl-dilaurylammonium, le chlorure de méthyltrioctylammonium, le chlorure de méthyl-tridécylammonium, le chlorure de benzyl-diméthyl-cétylammonium. Les bromures sont les sels préférés. Les sels d'ammonium quaternaire peuvent être mis en oeuvre tels quels ou sous forme d'une solution dans un milieu hydrocarboné choisi dans le groupe formé par les hydrocarbures et / ou par le butène-1 produit de dimérisation et / ou par un, ou les, sous-produit de la réaction tels que les hexènes.

Que ce soit un procédé continu ou discontinu, les sels d'ammonium quaternaire, purs ou en solution, peuvent être introduits avant de procéder à la réaction de dimérisation de l'éthylène, par exemple ils peuvent être utilisés pour effectuer un traitement de passivation des parois de l'enceinte réactionnelle préalablement au démarrage de la réaction. Les parois de l'enceinte sont métalliques (métaux, aciers, alliages,...) et peuvent avoir subi des traitements de protection (polissage, vitrification,...) ou peuvent avoir été soumises à une protection anodique.

La passivation est effectuée selon toutes les techniques connues. Avantageusement, on charge l'enceinte avec une solution de 20 ppm à 5 % en poids d'additif dans un milieu hydrocarboné, le contact est maintenu de préférence sous agitation pendant 10 min à 10 h, de préférence 30 min à 3 h, à une température inférieure à la température d'ébullition du solvant, 20 à 100° C généralement et 30 à 80°C de préférence. La solution est ensuite généralement évacuée.

Les sels d'ammonium quaternaire, purs ou en solution, peuvent aussi être introduits de façon continue ou en discontinu pendant le déroulement de la réaction, par exemple en mélange avec la solution du titanate, de préférence sous forme d'un flux indépendant des flux de catalyseur. Il peut être avantageux de combiner un traitement de passivation préalable de l'enceinte réactionnelle suivi d'une injection en continu ou en discontinu pendant le déroulement de la réaction.

La quantité de sels d'ammonium quaternaire mise en oeuvre pendant la réaction de dimérisation peut représenter de 1 partie par million en poids (ppm) à 5 % en poids, avantageusement 1 ppm à 1 %, de préférence de 20 ppm à 5000 ppm, par rapport au butène-1 produit, que cette quantité soit introduite pendant la réaction (procédé continu) ou dans l'enceinte avant réaction (procédé discontinu).

La réaction de dimérisation de l'éthylène peut être mise en oeuvre à une température de 20 à 150° C, de préférence de 20 à 70° C et de manière encore plus préférée de 40 à 70° C. La pression est de préférence de 0,5 à 8 MPa.

Dans un mode de mise en oeuvre de la réaction catalytique de dimérisation en discontinu, on introduit dans un réacteur (enceinte réactionnelle) muni des habituels systèmes d'agitation et de refroidissement l'additif avec la solution de catalyseur, par exemple un volume choisi de solution catalytique, préparée comme décrit ci-dessus, et, indépendamment, un volume choisi d'une solution de sel d'ammonium quaternaire, après quoi on pressurise par de l'éthylène et on ajuste la température à la valeur souhaitée. On alimente le réacteur par de l'éthylène à pression constante jusqu'à ce que le volume total de liquide produit remplisse presque complètement le réacteur. On détruit après réaction le catalyseur, par exemple par injection d'eau, d'ammoniac ou d'une amine, et on soutire et sépare les produits de la réaction et les solvants éventuels.

En cas d'opération en continu, on peut avantageusement commencer chaque marche par une passivation des parois du réacteur par un volume choisi d'une solution de sel d'ammonium quaternaire. Après avoir soutiré cette solution et avantageusement rincé le réacteur avec un hydrocarbure, la solution catalytique est injectée en continu en même temps qu'une solution de sel d'ammonium quaternaire et en même temps que l'éthylène. La température et la pression sont maintenues constantes au moyen de tout système de régulation habituel. L'effluent du réacteur est envoyé dans un système de colonnes à distiller qui permet de séparer d'une part le butène-1 de l'éthylène, qui est renvoyé au réacteur, d'autre part les hexènes et les octènes sous-produits, dont une partie peut être renvoyée dans la section de préparation du catalyseur. Le pied de colonne contenant le catalyseur, les sous-produits lourds et l'additif peut-être incinéré, ou bien le catalyseur récupéré est recyclé.

Les exemples suivants illustrent l'invention sans en limiter la portée.

### EXEMPLES 1 A 4

Une série de tests a été effectuée pour déterminer l'effet inhibiteur des sels d'ammonium quaternaire sur la formation et l'adhérence aux parois du polymère sous-produit dans la dimérisation de l'éthylène en butène-1. L'effet de la production de polymère est très important en termes d'encrassement des parois du réacteur et des tubes des échangeurs parce qu'il limite les transferts de chaleur indispensables à l'élimination de la chaleur de réaction. Cet effet néfaste est observé même quand la quantité d'éthylène transformé en polymère est très faible en regard de la quantité d'éthylène qui est dimérisé en butène-1.

On utilise pour effectuer la réaction de dimérisation de l'éthylène un autoclave de type Grignard en acier inoxydable d'un volume de 250 ml, muni d'une double enveloppe permettant de réguler la température par une circulation d'eau, et d'un barreau magnétique pour l'agitation.

Dans chaque exemple, le catalyseur est préparé, à une température de 25 °C, en introduisant successivement dans le réacteur sous pression atmosphérique d'éthylène : 25 ml d'heptane, 9,8 ml d'une solution de 0,5 ml de triéthylaluminium dans 19,5 ml d'heptane (soit 1,77 mmole), 2 ml d'une solution à 10 % en volume de titanate de tétra-n-butyle dans l'heptane (soit 0,59 mmole), et une quantité variable selon les essais de l'additif sel d'ammonium quaternaire. Après 2 minutes d'interaction, la température est portée à 70 °C et la pression d'éthylène à 2 MPa.

La réaction de dimérisation est arrêtée par injection de 2 ml d'eau lorsque environ 100 litres gazeux (ramenés aux conditions normales) d'éthylène ont été consommés. Le réacteur est ensuite dépressurisé, le gaz étant comptabilisé et recueilli dans un gazomètre pour être analysé. Après ouverture du réacteur, on en collecte le contenu liquide et solide, qui est lavé avec 20 ml d'une solution aqueuse sulfurique à 10 % afin de redissoudre les résidus de catalyseur. Le polymère solide restant est filtré, séché une nuit à l'étuve à 110 °C et pesé.

Les résultats des tests sont présentés dans le tableau 1, où figurent pour chaque essai : la nature de l'additif, la quantité d'additif mis en oeuvre exprimée en parties par million en poids (ppm) par rapport au butène-1 formé, l'activité du catalyseur exprimée par le nombre de moles d'éthylène consommées par heure, la quantité de polymère formé en ppm par rapport à l'éthylène transformé, et l'aspect physique de ce polymère.

L'exemple 1 est un exemple comparatif en l'absence d'additif, qui ne fait pas partie de l'invention. Par comparaison, il est clair que la mise en oeuvre d'un additif constitué par un sel d'ammonium quaternaire a un effet tout à fait bénéfique sur la quantité de polymère formé d'une part, qui est fortement diminuée, et sur son adhérence aux parois d'autre part, qui est bien plus faible, ce qui facilite son élimination.

**TABLEAU 1**

| N° | Additif | Additif / Butène-1 (ppm) | Activité (mol C₂ / h) | Polymère (ppm) | Aspect |
|---|---|---|---|---|---|
| 1 | sans | 0 | 6,0 | 2330 | (a) |
| 2 | (C₂H₅)₄N⁺Cl⁻ | 330 | 4,1 | 1450 | (b) |
| 3 | (C₂H₅)₄N⁺Br⁻ | 400 | 4,2 | 430 | (b) |
| 4 | [(CH₃)₃(C₁₆H₃₃)]N⁺Br⁻ | 850 | 4,0 | 230 | (b) |

| | | | | | |
|---|---|---|---|---|---|
| (a) polymère très fortement collé aux parois, | | | | | |
| (b) polymère en granules d'adhérence très faible. | | | | | |

## Revendications

1. Procédé de conversion de l'éthylène en butène-1, dans lequel, dans une enceinte réactionnelle, on met l'éthylène en contact avec une solution d'un catalyseur résultant de l'interaction d'au moins un titanate d'alkyle avec au moins un composé d'aluminium de formule AlR₃ ou AlR₂H, chacun des restes R étant un radical hydrocarbyle, procédé caractérisé en ce qu'il se déroule en présence d'au moins un additif choisi dans le groupe formé par les sels d'ammonium quaternaire.

2. Procédé selon la revendication 1, caractérisé en ce que la solution de catalyseur résulte de l'interaction d'un mélange préformé d'au moins un titanate d'alkyle et d'au moins un éther, avec au moins un composé d'aluminium de formule AlR₃ ou AlR₂H, chacun des restes R étant un radical hydrocarbyle.

3. Procédé selon l'une des revendications précédentes, caractérisé en ce que la concentration du titane dans la solution de catalyseur est comprise entre 10⁻⁴ et 0,5 mole par litre.

4. Procédé selon l'une des revendications précédentes, caractérisé en ce que le rapport molaire entre le composé de l'aluminium et celui du titane est compris entre 1:1 et 20:1.

5. Procédé selon l'une des revendications 2 à 4, caractérisé en ce que le rapport molaire entre l'éther et le composé du titane est inférieur ou égal à 10.

6. Procédé selon l'une des revendications précédentes, caractérisé en ce que le composé de l'aluminium est AlR₃.

7. Procédé selon l'une des revendications précédentes, caractérisé en ce que le sel d'ammonium quaternaire est choisi dans le groupe formé par les halogénures ou les hydroxydes d'ammonium quaternaire.

8. Procédé selon l'une des revendications précédentes, caractérisé en ce que l'additif est choisi dans le groupe formé par les bromures d'ammonium quaternaire.

9. Procédé selon l'une des revendications précédentes, caractérisé en ce que l'ammonium quaternaire comporte des radicaux hydrocarbyle, identiques ou différents, par exemple des radicaux alkyle, cycloalkyle, aryle, cycloalkyle ou aryle substitués par un groupe alkyle, comprenant de 1 à 30 atomes de carbone.

10. Procédé selon l'une des revendications précédentes, caractérisé en ce que l'additif est mis en oeuvre sous forme d'une solution dans un milieu hydrocarboné choisi dans le groupe formé par les hydrocarbures, le produit de dimérisation, le ou les sous-produits de réaction, et leurs mélanges.

11. Procédé selon l'une des revendications 1 à 9, caractérisé en ce que l'additif est mis en oeuvre à l'état pur.

12. Procédé selon l'une des revendications précédentes, caractérisé en ce que, avant de procéder à la conversion, l'additif est introduit dans l'enceinte réactionnelle pour effectuer un traitement de passivation des parois de l'enceinte.

13. Procédé selon l'une des revendications précédentes, caractérisé en ce que l'additif est introduit pendant le déroulement de la réaction de conversion, le procédé étant continu.

14. Procédé selon l'une des revendications 1 à 12, caractérisé en ce que, le procédé étant discontinu, l'additif est introduit dans l'enceinte réactionnelle avec la solution de catalyseur.

15. Procédé selon l'une des revendications précédentes, caractérisé en ce que l'additif est introduit indépendamment de la solution de catalyseur.

16. Procédé selon l'une des revendications précédentes, caractérisé en ce que la quantité de sel d'ammonium quaternaire mise en oeuvre pendant la réaction de conversion est de 1 ppm à 5 % en poids par rapport au butène-1 produit.

17. Procédé selon l'une des revendications précédentes, caractérisé en ce que la quantité de sel d'ammonium quaternaire mise en oeuvre pendant la réaction de conversion est de 20 ppm à 5000 ppm par rapport au butène-1 produit.

18. Procédé selon l'une des revendications précédentes, caractérisé en ce que le procédé se déroule à une température comprise entre 20 et 150 °C sous une pression de 0,5 à 8 MPa.

19. Procédé selon l'une des revendications précédentes, caractérisé en ce que l'additif est choisi dans le groupe formé par le chlorure de tétraéthylammonium, le bromure de tétraéthylammonium, le chlorure de triméthyl-cétylammonium, le bromure de triméthyl-cétylammonium, le chlorure de diméthyl-dilaurylammonium, le chlorure de méthyltrioctylammonium, le chlorure de méthyl-tridécylammonium, le chlorure de benzyl-diméthyl-cétylammonium.

## Patentansprüche

1. Verfahren zur Umwandlung von Ethylen in Buten-1, bei dem man in einem Reaktionsgefäß das Ethylen mit einer Lösung eines Katalysators kontaktiert, die aus der Wechselwirkung wenigstens eines Alkyltitanats mit wenigstens einer Aluminiumverbindung der Formel ALR₃ oder ALR₂H resultiert, wobei jeder der Reste R ein Hydrocarbylradikal ist, dadurch gekennzeichnet, daß es in Anwesenheit wenigstens eines Additives, das gewählt ist aus der durch diequaternären Ammoniumsalze gebildeten Gruppe, abläuft.

2. Verfahren nach Anspruch 1, dadurch gekennzeichnet, daß die Katalysatorlösung aus der Wechselwirkung eines vorgeformten Gemisches wenigstens eines Alkyltitanats und wenigstens eines Äthers mit wenigstens einer Aluminiumverbindung der Formel ALR₃ oder ALR₂H resultiert, wobei jeder der Reste R ein Hydrocarbylradikal ist.

3. Verfahren nach einem der vorhergehenden Ansprüche, dadurch gekennzeichnet, daß die Konzentration des Titanats in der Katalysatorlösung zwischen 10⁻⁴ und 0,5 Mol/l liegt.

4. Verfahren nach einem der vorhergehenden Ansprüche, dadurch gekennzeichnet, daß das Mol-Verhältnis zwischen der Verbindung des Aluminiums und der des Titatanats zwischen 1:1 und 20:1 beträgt.

5. Verfahren nach einem der Ansprüche 2 bis 4, dadurch gekennzeichnet, daß das Mol-Verhältnis zwischen dem Äther und der Titanverbindung kleiner oder gleich 10 ist.

6. Verfahren nach einem der vorhergehenden Ansprüche, dadurch gekennzeichnet, daß die Verbindung des Aluminiums ALR₃ ist.

7. Verfahren nach einem der vorhergehenden Ansprüche, dadurch gekennzeichnet, daß das quaternäre Amoniumsalz gewählt ist aus der durch die Halogenide oder die Hydroxyde des quatemären Ammoniums gebildeten Gruppe.

8. Verfahren nach einem der vorhergehenden Ansprüche, dadurch gekennzeichnet, daß das Additiv gewählt ist aus der durch die Bromide des quaternären Ammoniums gebildeten Gruppe.

9. Verfahren nach einem der vorhergehenden Ansprüche, dadurch gekennzeichnet, daß das quaternäre Ammonium identische oder unterschiedliche Hydrocarbylradikale, beispielsweise Alkyl-, Cycloalkyl-, Aryl-, Cycloalkyl- oder durch eine Alkylgruppe substituierte Arylradikale umfaßt, , die 1 bis 3 Kohlenstoffatome umfassen.

10. Verfahren nach einem der vorhergehenden Ansprüche, dadurch gekennzeichnet, daß das Additiv in Form einer Lösung in einer kohlenstoffhaltigen Verbindung eingesetzt wird, die gewählt ist aus der durch die Kohlenwasserstoffe, das Dimerisierungsprodukt, sowie das oder die Reaktionsnebenprodukte und deren Gemische gebildeten Gruppe.

11. Verfahren nach einem der Ansprüche 1 bis 9, dadurch gekennzeichnet, daß das eingesetzte Additiv sich im reinen Zustand befindet.

12. Verfahren nach einem der vorhergehenden Ansprüche, dadurch gekennzeichnet, daß bevor die Umwandlung in Angriff genommen wird, das Additiv in das Reaktionsgefäß eingeführt wird, um eine Passivierungsbehandlung der Wandungen des Gefäßes vorzunehmen.

13. Verfahren nach einem der vorhergehenden Ansprüche, dadurch gekennzeichnet, daß das Additiv während des Ablaufs der Umwandlungsreaktion eingeführt wird, wobei das Verfahren kontinuierlich durchgeführt wird.

14. Verfahren nach einem der Ansprüche 1 bis 12, dadurch gekennzeichnet, daß das Verfahren diskontinuierlich durchgeführt wird, wobei das Additiv ins das Reaktionsgefäß mit der Katalysatorlösung eingeführt wird.

15. Verfahren nach einem der vorhergehenden Ansprüche, dadurch gekennzeichnet, daß das Additiv unabhängig von der Katalysatorlösung eingeführt wird.

16. Verfahren nach einem der vorhergehenden Ansprüche, dadurch gekennzeichnet, daß die Menge an während der Umwandlungsreaktion eingesetztem im quaternären Ammoniumsalz zwischen 1 ppm und5 Gewichtsprozent bezogen auf das erzeugte Buten-1 liegt.

17. Verfahren nach einem der vorhergehenden Ansprüche, dadurch gekennzeichnet, daß die Menge des während der Umwandlungsreaktion eingesetzten quaternären Ammoniumsalzes bei 20 ppm bis 5000 ppm bezogen auf das erzeugte Buten-1 liegt.

18. Verfahren nach einem der vorhergehenden Ansprüche, dadurch gekennzeichnet, daß das Verfahren bei einer Temperatur zwischen 20 und 150 °C unter einem Druck von 0,5 bis 8 Mpa abläuft.

19. Verfahren nach einem der vorhergehendenAnsprüche, dadurch gekennnzeichnet, daß das Additiv gewählt ist aus der Gruppe, die gebildet wird durch Ammoniumtetraethylchlorid, Ammoniumtetraethylbromid, Ammoniumtrimethylcetylchlorid, Ammoniumtrimethyl-cetylbromid, Ammoniumdimethyl-diarylchlorid, Ammoniummethyltrioktylchlorid, Ammoniummethyltridecylchlorid und Ammoniumbenzyl-dimethyl-cetylchlorid.

## Claims

1. A process for the conversion of ethylene to but-1-ene, wherein, in a reaction enclosure, the ethylene is brought into contact with a solution of a catalyst resulting from the interaction of at least one alkyl titanate with at least one aluminium compound of the formula AlR₃ or AlR₂H, each of the residues R being a hydrocarbyl radical, the process being characterised in that it takes place in the presence of at least one additive selected from the group formed by quaternary ammonium salts.

2. A process according to claim 1 characterised in that the catalyst solution results from the interaction of a preformed mixture of at least one alkyl titanate and at least one ether, with at least one aluminium compound of the formula AlR₃ or AlR₂H, each of the residues R being a hydrocarbyl radical.

3. A process according to one of the preceding claims characterised in that the concentration of titanium in the catalyst solution is between 10⁻⁴ 4 and 0.5 mole per litre.

4. A process according to one of the preceding claims characterised in that the molar ratio between the aluminium compound and the titanium compound is between 1:1 and 20:1.

5. A process according to one of claims 2 to 4 characterised in that the molar ratio between the ether and the titanium compound is less than or equal to 10.

6. A process according to one of the preceding claims characterised in that the aluminium compound is AlR₃.

7. A process according to one of the preceding claims characterised in that the quaternary ammonium salt is selected from the group formed by quaternary ammonium hydroxides or halides.

8. A process according to one of the preceding claims characterised in that the additive is selected from the group formed by quaternary ammonium bromides.

9. A process according to one of the preceding claims characterised in that the quaternary ammonium comprises identical or different hydrocarbyl radicals, for example alkyl, cycloalkyl, aryl, cycloalkyl or aryl which are substituted by an alkyl group, comprising from 1 to 30 carbon atoms.

10. A process according to one of the preceding claims characterised in that the additive is used in the form of a solution in a hydrocarbon medium selected from the group formed by hydrocarbons, the dimerisation product, the reaction by-product or by-products and mixtures thereof.

11. A process according to one of claims 1 to 9 characterised in that the additive is used in the pure state.

12. A process according to one of the preceding claims characterised in that, before proceeding with the conversion operation, the additive is introduced into the reaction enclosure to effect a treatment for passivation of the walls of the enclosure.

13. A process according to one of the preceding claims characterised in that the additive is introduced while the conversion reaction is taking place, the process being continuous.

14. A process according to one of claims 1 to 12 characterised in that, the process being discontinuous, the additive is introduced into the reaction enclosure with the catalyst solution.

15. A process according to one of the preceding claims characterised in that the additive is introduced independently of the catalyst solution.

16. A process according to one of the preceding claims characterised in that the amount of quaternary ammonium salt used during the conversion reaction is from 1 ppm to 5% by weight with respect to the but-1-ene produced.

17. A process according to one of the preceding claims characterised in that the amount of quaternary ammonium salt used during the conversion reaction is from 20 ppm to 5000 ppm with respect to the but-1-ene produced.

18. A process according to one of the preceding claims characterised in that the process takes place at a temperature of between 20 and 150°C under a pressure of from 0.5 to 8 MPa.

19. A process according to one of the preceding claims characterised in that the additive is selected from the group formed by tetraethyammonium chloride, tetraethylammonium bromide, trimethyl-cetylammonium chloride, trimethyl-cetylamnoniun bromide, dimethyl-dilaurylammonium chloride, methyl-trioctylammonium chloride, methyl-tridecylammonium chloride and benzyl-dimethyl-cetylammonium chloride.
